(19) Europäisches Patentamt
European Patent Office
Office européen des brevets

(11) Numéro de publication : **0 420 722 B1**

(12) # FASCICULE DE BREVET EUROPEEN

(45) Date de publication du fascicule du brevet : **23.02.94 Bulletin 94/08**

(51) Int. Cl.$^5$ : **C07C 271/16,** A61K 7/48, A61K 31/27

(21) Numéro de dépôt : **90402577.2**

(22) Date de dépôt : **19.09.90**

(54) **Nouveaux composés lipidiques dérivés des sphingosines, leur procédé de préparation et leurs applications, notamment en cosmétique et en dermopharmacie.**

(30) Priorité : **21.09.89 FR 8912423**

(43) Date de publication de la demande : **03.04.91 Bulletin 91/14**

(45) Mention de la délivrance du brevet : **23.02.94 Bulletin 94/08**

(84) Etats contractants désignés : **AT BE CH DE DK FR GB IT LI NL SE**

(56) Documents cités :
**EP-A- 0 097 059**
**HELVETICA CHIMICA ACTA, vol. 71, 1988, pages 254-361, Basel, CH; P. HEROLD: "39. Synthesis of D-erythro and D-threo-sphingosine derivatives from L-serine"**

(73) Titulaire : **L'OREAL**
**14, Rue Royale**
**F-75008 Paris (FR)**

(72) Inventeur : **Zysman, Alexandre**
**6, rue Georges Eastman**
**F-75013 Paris (FR)**
Inventeur : **Vanlerberghe, Guy**
**40, rue du Général de Gaulle**
**Villevaudé, F-77410 Claye-Souilly (FR)**
Inventeur : **Sebag, Henri**
**26, rue Erlanger**
**F-75016 Paris (FR)**

(74) Mandataire : **Casalonga, Axel et al**
**BUREAU D.A. CASALONGA - JOSSE**
**Morassistrasse 8**
**D-80469 München (DE)**

**EP 0 420 722 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux composés lipidiques dérivés des sphingosines, leur procédé de préparation, ainsi que leur utilisation, notamment pour les traitements et les soins de la peau et des cheveux en cosmétique ou en dermopharmacie.

L'exposition de la peau au froid, au soleil, aux atmosphères à faible humidité relative, les traitements répétés avec des compositions de lavage ou encore le contact avec des solvants organiques, sont des facteurs qui entraînent, à des degrés divers, un dessèchement apparent. La peau apparaît plus sèche, moins souple et le relief cutané plus prononcé.

Par ailleurs, les cheveux, qui sont soumis trop fréquemment à certains traitements capillaires, perdent leur aspect brillant et peuvent devenir rèches et cassants.

La demanderesse a donc recherché des composés qui permettent de prévenir ou de corriger ces phénomènes se traduisant par un dessèchement apparent et qui redonnent à la peau sa souplesse et son relief et aux cheveux leur brillance et leur douceur.

C'est ainsi que la demanderesse a découvert de nouveaux composés dont la constitution peut être représentée par la formule suivante :

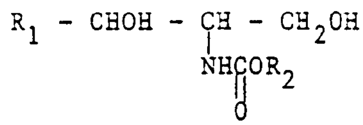

(I)

dans laquelle :

. $R_1$ désigne un radical alcoyle ou alcényle en $C_{11}$ à $C_{21}$ portant éventuellement un groupement hydroxyle;

. $R_2$ désigne un radical hydrocarboné en $C_8$ à $C_{30}$, linéaire ou ramifié, saturé ou bien portant une ou plusieurs insaturations éthyléniques, et désigne de préférence un radical alcoyle ou alcényle.

Les composés lipidiques (I) ci-dessus sont, par leur structure, très proches des céramides qui sont les éléments constitutifs prépondérants des lipides intercornéocytaires du stratum corneum. On admet généralement qu'ils participent au maintien de l'intégrité de la barrière cutanée. On en trouve également à moindre degré, au niveau du cheveu. Les composés (I) selon l'invention, qui sont des cires de bas point de fusion, présentent donc un intérêt tout particulier pour les traitements et les soins de la peau et des cheveux en cosmétique ou en dermopharmacie en permettant de prévenir ou de corriger certains effets du dessèchement apparent.

Ces composés présentent par ailleurs une faible agressivité vis-à-vis de la peau ou des muqueuses oculaires et une bonne tolérance vis-à-vis des membranes cellulaires comme celles des érythrocytes.

Les nouveaux composés de formule (I) ci-dessus présentent des propriétés émollientes et adoucissantes. Ils sont facilement solubilisés dans les phases grasses des préparations cosmétiques ou dermopharmaceutiques.

Les composés de formule (I) dans laquelle $R_2$ représente une chaîne linéaire, qu'on appellera ci-après composés (I'), forment, en association avec d'autres lipides, des vésicules.

La présente invention a ainsi pour objet les nouveaux composés lipidiques de formule (I) définis ci-dessus.

Les composés lipidiques de formule (I) ci-dessus résultent de la condensation, sur la fonction amine d'une sphingosine, d'une dihydrosphingosine ou d'une phytosphingosine, d'un alcoyl- ou alcénylchloroformiate ou d'un alcoyl- ou alcénylimidazolide.

Un autre objet de la présente invention est donc constitué par le procédé de préparation des composés de formule (I) qui peut être représenté par le schéma suivant :

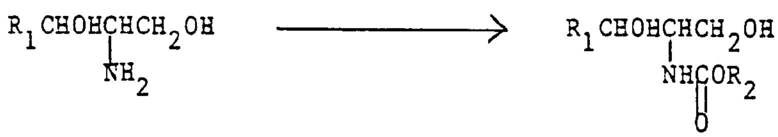

(II) (I)

$R_1$ et $R_2$ ayant les significations indiquées ci-dessus.

Les composés (I) sont obtenus par réaction des composés de formule (II) soit avec un alcoyl- ou alcénylchloroformiate dans des solvants tels que le diméthylformamide ou le tétrahydrofuranne en présence d'une base comme par exemple la triéthylamine, la pyridine etc., soit avec un alcoyl- ou alcénylimidazolide isolé ou

préparé in situ par exemple par réaction d'un alcool gras avec le carbonyldiimidazole.

Les alcoyl- ou alcénylchloroformiates sont des produits commerciaux.

Les alcoyl- ou alcénylimidazolides préparés in situ ou isolés à l'état pur sont synthétisés suivant les méthodes décrites par H.A. STAAB dans Angew. Chem. International Edit. vol. 1 (1962), n° 7, p. 351.

Pour la préparation du composé (I) de l'invention, on peut également utiliser le chlorhydrate du composé (II). Ce chlorhydrate est alors solubilisé de préférence dans le tétrahydrofuranne additionné de 10% d'eau. La base (II) est alors libérée in situ par un excès d'hydrogénocarbonate.

Les composés (II) sont des composés connus. Leur synthèse a été décrite en particulier par D. SHAPIRO dans "Chemistry of sphingolipids", HERMANN, Paris (1969).

Quand $R_1$ désigne un radical alcényle, les composés (II) sont des sphingosines.

Quand $R_1$ désigne un radical alcoyle, les composés (II) sont des dihydrosphingosines. Elles peuvent être préparées en particulier partir de 2-acétamido-3-oxo-alcanoate de méthyle ou d'éthyle, comme décrit dans "Chemistry of sphingolipids" p. 32.

Quand $R_1$ désigne un radical alcoyle hydroxylé, les composés (II) sont des phytosphingosines. Elles peuvent être préparées entre autres à partir des sphingosines suivant le schéma décrit par B. Weiss dans Biochemistry, 4, p. 686 (1965).

Les procédés de synthèse des sphingosines, dihydrosphingosines, et phytosphingosines décrits ci-dessus conduisent à des mélanges racémiques.

Il est possible d'obtenir ces composés sous la forme d'énantiomères purs en faisant une résolution du racémique décrite par exemple par SHAPIRO dans l'article précité, à la page 99.

On peut également synthétiser directement des énantiomères purs. De très nombreux procédés ont été décrits comme par exemple par R. SCHMIDT dans "Tetrahedron Letters", vol. 27, n° 4, pages 481-84 (1986), par B. BERNET dans la même revue, vol. 24, n° 49, pages 5491-5494 (1983) ou encore par MAKOTO KISO dans "Carbohydrate Research", 158 (1986), pages 101-111 etc.

Certains composés (II) sont des composés du commerce, comme la D-sphingosine vendue par la société Sigma.

Les composés selon l'invention peuvent recevoir des applications diverses, notamment en tant que constituants cireux dans des compositions cosmétiques et dermopharmaceutiques. Les composés de formule (I') possèdent en plus la propriété de former des vésicules en association avec d'autres lipides, lorsqu'ils sont dispersés dans l'eau.

La présente invention a donc pour objet l'utilisation des composés lipidiques de formule (I) en tant que constituants cireux dans des émulsions, des dispersions ou dans des lotions. Elle a également pour objet l'utilisation des composés de formule (I'), associés à d'autres lipides, pour la formation de sphérules lipidiques.

La présente invention a également pour objet des compositions à usage cosmétique ou dermopharmaceutique contenant un composé de formule (I).

Les compositions selon l'invention peuvent se présenter sous forme d'émulsions (lait ou crème), de lotions hydroalcooliques, huileuses ou oléoalcooliques, de gels, de dispersions ou de bâtonnets solides.

Selon l'invention, les composés de formule (I) représentent 0,05% à 20%, et de préférence 0,1 à 10% du poids total de la composition.

Les compositions sont par exemple des lotions, des laits ou des crèmes émollients, des lotions, des laits ou des crèmes pour les soins de la peau ou des cheveux, des crèmes ou des laits démaquillants, des bases de fond de teint, des lotions, des laits ou des crèmes antisolaires, des lotions, des laits ou des crèmes de bronzage artificiel, des crèmes ou des mousses de rasage, des lotions après rasage.

Ces compositions peuvent également se présenter sous la forme de bâtons pour les lèvres destinés soit à les colorer, soit à éviter les gerçures, ou de produits de maquillage pour les yeux ou de fards et fonds de teint pour le visage.

Lorsque les compositions selon l'invention se présentent sous forme d'émulsions du type eau-dans-l'huile ou huile-dans-l'eau, la phase grasse est essentiellement constituée d'un mélange de composé de formule (I) avec au moins une huile, et éventuellement un autre corps gras.

La phase grasse des émulsions peut constituer 5 à 60% du poids total de l'émulsion.

La phase aqueuse des dites émulsions constitue de préférence 30 à 85% du poids total de l'émulsion.

La proportion de l'agent émulsionnant peut être comprise entre 1 et 20%, et de préférence entre 2 et 12% du poids total de l'émulsion.

Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau ou les cheveux; les lotions huileuses peuvent en outre constituer des huiles moussantes contenant un tensio-actif oléosoluble, des huiles pour le bain, etc.

Parmi les principaux adjuvants pouvant être présents dans les compositions selon l'invention, on peut citer

les corps gras tels que les huiles ou les cires minérales, animales ou végétales, les acides gras, les esters d'acides gras tels que les triglycérides d'acides gras ayant de 6 à 18 atomes de carbone, les alcools gras; des émulsionnants comme les alcools gras oxyéthylénés ou les alcoyléthers de polyglycérol; des solvants tels que les monoalcools ou polyalcools inférieurs contenant de 1 à 6 atomes de carbone ou encore l'eau.

Les mono- ou polyalcools plus particulièrement préférés sont choisis parmi l'éthanol, l'isopropanol, le propylèneglycol, le glycérol et le sorbitol.

A titre de corps gras, parmi les huiles minérales, on peut citer l'huile de vaseline; parmi les huiles animales, les huiles de baleine, de phoque, de menhaden, de foie de flétan, de morue, de thon, de tortue, de pied de boeuf, de pied de cheval, de pied de mouton, de vison, de loutre, de marmotte, etc.; parmi les huiles végétales, les huiles d'amande, d'arachide, de germe de blé, d'olive, de germe de maïs, de jojoba, de sésame, de tournesol, de palme, de noix, de karité, de shoréa, de macadamia, de pépins de cassis et similaires.

Parmi les esters d'acides gras, on peut utiliser des esters d'acides en $C_{12}$ à $C_{22}$ saturés ou insaturés et d'alcools inférieurs comme l'isopropanol ou le glycérol ou d'alcools gras en $C_8$ à $C_{22}$, linéaires ou ramifiés, saturés ou insaturés ou encore d'alcanediols-1,2 en $C_{10}$-$C_{22}$.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de candellila, la cire microcristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de Ca, Mg et Al.

Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de polyglycérol, les alcools en $C_{12}$-$C_{18}$ comportant de 2 à 10 moles de glycérol.

Il peut aussi être utile d'utiliser des épaississants tels que les dérivés de cellulose, les dérivés d'acide polyacrylique, les gommes de guar ou de caroube ou la gomme de xanthane.

La composition selon l'invention peut également contenir des adjuvants habituellement utilisés en cosmétique ou en dermopharmacie et notamment des produits hydratants, des adoucissants, des produits pour le traitement d'affections cutanées, des filtres solaires, des germicides, des colorants, des conservateurs, des parfums et des propulseurs.

Lorsque les compositions selon l'invention sont des dispersions, il peut s'agir de dispersions de composés de formule (I) dans l'eau en présence de tensio-actif ou encore de dispersions aqueuses de sphérules lipidiques, constituées de couches moléculaires organisées enfermant une phase aqueuse encapsulée, ces couches étant constituées d'au moins un composé de formule (I'), associé à au moins un autre composé lipidique.

On peut citer, à cet effet, comme composé lipidique, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate de sodium, les alkylsulfates tels que le cétylsulfate de sodium, ou encore les lipides du type de ceux décrits dans les brevets français n° 2 315 991, 1 477 048 et 2 091 516 ou dans la demande de brevet internationale WO 83/01 571.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tetradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. A titre de groupement ionique, on peut avantageusement utiliser un groupement dérivé d'un composé amphotère, anionique ou cationique. On peut aussi utiliser avantageusement comme lipides formant la phase lamellaire, des éthers de polyglycérol tels que ceux décrits dans les brevets français n° 1 477 048, 2 091 516, 2 465 780 et 2 482 128.

D'autres lipides décrits dans la demande de brevet internationale WO 83/01 571 comme pouvant être utilisés pour la formation de liposomes sont les glycolipides comme le lactosylcéramide, le galactocérébroside, les gangliosides et le trihexosylcéramide, ainsi que les phospholipides tels que le phosphatidylglycérol et le phosphatidylinositol.

La présente invention a donc également pour objet une dispersion de sphérules lipidiques constituées de couches moléculaires organisées de composé(s) de formule (I') et de lipide défini ci-dessus renfermant une phase aqueuse à encapsuler.

La phase continue de la dispersion qui entoure les sphérules est une phase aqueuse.

Les sphérules en dispersion ont un diamètre compris entre 0,1 μm et 5 μm.

La phase aqueuse encapsulée dans les sphérules peut être de l'eau ou une solution aqueuse de substance active et est dans ce cas de préférence isoosmotique par rapport à la phase continue de la dispersion.

Les sphérules peuvent être obtenues en particulier suivant le procédé décrit dans le brevet français 2 315 991 de la demanderesse, selon lequel on prépare une dispersion de sphérules constituées de couches moléculaires organisées renfermant une phase aqueuse à encapsuler, en mettant en contact, d'une part un ou plusieurs composé(s) lipidique(s) de formule (I') associé(s) à un ou plusieurs lipides défini(s) ci-dessus, et d'autre part la phase aqueuse à encapsuler dans les sphérules, en agitant pour assurer le mélange et obtenir une phase lamellaire, en ajoutant ensuite un liquide de dispersion en quantité supérieure à la quantité de phase lamellaire obtenue et en secouant énergiquement pendant une durée allant de 15 minutes à 3 heures environ.

Le rapport pondéral entre la phase aqueuse à encapsuler et le(s) composé(s) de formule (I') associé aux lipides formant la phase lamellaire, est de préférence compris entre 0,1 et 3.

Le rapport pondéral de la phase aqueuse de dispersion que l'on ajoute à la phase lamellaire que l'on disperse, est de préférence compris entre 2 et 100, la phase de dispersion et la phase aqueuse à encapsuler étant de préférence isoosmotiques.

L'agitation est réalisée au moyen d'un agitateur à secousses. Le procédé est de préférence mis en oeuvre à une température comprise entre 30° et 120°C.

Un autre procédé de préparation peut consister à utiliser le procédé dénommé REV (reverse-phase évaporation vesicle) ou évaporation en phase inverse décrit dans Proc. Natl. Acad. Sci. USA., Vol. 75, n° 9, pages 4194-4198 (1978); par SZOKA et PAPAHADJOPOULOS.

Les substances actives pouvant être encapsulées dans la phase aqueuse peuvent être des substances ayant un intérêt pharmaceutique, alimentaire ou des substances ayant une activité cosmétique.

Les substances ayant une activité cosmétique peuvent être des produits destinés aux soins de la peau et du cheveu tels que par exemple des humectants comme la glycérine, le sorbitol, le pentaérythritol, l'inositol, l'acide pyrrolidone carboxylique et ses sels; des agents de brunissage artificiel tels que la dihydroxy-acétone, l'érythrulose, le glycéraldéhyde, les γ-dialdéhydes tels que l'aldéhyde tartrique, ces composés étant éventuellement associés à des colorants; des filtres solaires hydrosolubles; des anti-perspirants, des déodorants, des astringents, des produits rafraîchissants, toniques, cicatrisants, kératolytiques, dépilatoires, des eaux parfumées; des extraits de tissus animaux ou végétaux, tels que les protéines, les polysaccharides, le liquide amniotique; des colorants hydrosolubles; des agents anti-pelliculaires; des agents antiséborrhéiques, des oxydants tels que des agents de décoloration comme l'eau oxygénée; des réducteurs tels que l'acide thioglycolique et ses sels.

Comme substances actives pharmaceutiques, on peut citer les vitamines, les hormones, les enzymes telles que le superoxyde dismutase, les vaccins, les anti-inflammatoires tels que l'hydrocortisone, les antibiotiques, les bactéricides, les agents cytotoxiques ou anti-tumoraux.

On peut également ajouter aux dispersions de sphérules selon l'invention divers adjuvants tels que des opacifiants, des gélifiants, des arômes, des parfums ou des colorants.

Les dispersions de sphérules lipidiques selon l'invention présentent l'intérêt de véhiculer des substances actives hydrophiles qui se trouvent ainsi masquées et protégées vis-à-vis des différents agents d'altération : oxydants et plus généralement composés réactifs vis-à-vis des substances actives encapsulées. La pénétration et la fixation des substances actives peuvent être modulées par la variation de la taille des sphérules et de leur charge électrique. L'action de ces substances actives peut également être ainsi différée (effet retard). Enfin, il est possible d'obtenir grâce à l'utilisation des lipides (I') selon l'invention, et de substances actives combinées, une action bénéfique spécifique de la substance active utilisée et en même temps assouplissante, particulièrement intéressante dans le cas du traitement de la peau et de la chevelure.

La présente invention a donc également pour objet l'utilisation en cosmétique d'une dispersion aqueuse de sphérules constituée de couches moléculaires organisées de composés lipidiques (I') associés à d'autres lipides renfermant une phase aqueuse à encapsuler, en particulier pour le traitement de la peau et des cheveux.

L'invention a également pour objet l'utilisation d'une telle dispersion de sphérules lipidiques en dermopharmacie ou dans l'industrie alimentaire.

La présente invention sera mieux illustrée par les exemples non limitatifs suivants.

EXEMPLE 1

1ère étape

Préparation du composé (II) avec : $R_1 = C_{15}H_{31}$ : chlorhydrate du 1,3-dihydroxy-2-amino-octadécane (mélange érythrothréo)

Le 2-acétamido-3-oxo octadécanoate de méthyle (100 g, soit 0,27 M) est mis en suspension dans 1 litre d'éthanol absolu. La température du milieu réactionnel est amenée en dessous de 0°C. A cette température, on ajoute en trois fois 30,7 g (0,8 M) de borohydrure de sodium et on maintient l'agitation à cette température pendant 3 heures. Le milieu réactionnel est alors porté à reflux du solvant pendant 3 heures. Après refroidissement à la température ordinaire, on ajoute 140cm³ d'acide chlorhydrique concentré et on porte à nouveau le milieu réactionnel à reflux pendant 3 heures. Ce milieu est filtré encore chaud sur un verre fritté. Le filtrat est concentré à sec sous pression réduite.

Le solide obtenu est recristallisé dans 300 cm³ de mélange de solvants heptane : acétate d'éthyle = 90 : 10. On isole 88 g d'un solide blanc dont l'indice d'acide mesuré dans l'éthanol par une solution de soude N/10 est de 2,99 meq/g.

Le spectre RMN¹³C de ce solide est conforme à la structure attendue

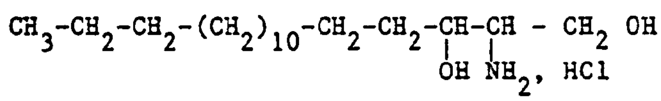

Il s'agit bien du chlorhydrate de dihydrosphingosine sous forme de mélange racémique érythro-thréo.

2ème étape

Préparation du composé (I) dans lequel $R_2$ = 2-éthylhexyle

Dans 110 cm³ de tétrahydrofuranne additionnés de 11cm³ d'eau, on solubilise à la température ambiante 20 g (5,92 x $10^{-2}$M) de chlorhydrate de dihydrosphingosine préparé ci-dessus.

A cette solution on ajoute 11, 41 g (5,92 x $10^{-2}$M) de 2-éthylhexylchloroformiate solubilisé dans 36cm³ de tétrahydrofuranne.

A ce mélange réactionnel on ajoute 20,11 g (0,237M) d'hydrogéno-carbonate de sodium et on l'abandonne à la température ambiante et sous agitation toute la nuit.

On ajoute ensuite 500cm³ d'eau. Après séparation des deux phases par décantation, on isole la phase supérieure et on élimine le solvant par distillation sous vide partiel. On recueille 22 g d'une cire de pF 50°C dont le spectre RMN¹³C confirme la structure attendue (mélange érythro-thréo) qui est la suivante :

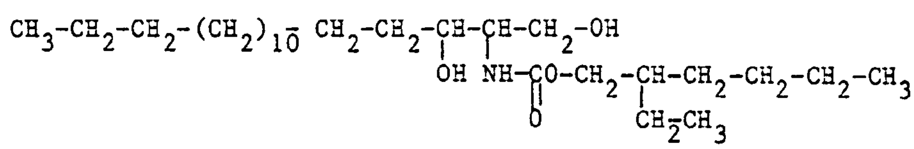

Analyse élémentaire :

| | Calculé | mesuré |
|---|---|---|
| C | 70,85 | 70,70 |
| H | 12,11 | 12,08 |
| N | 3,06 | 3,09 |

EXEMPLE 2

Préparation du composé (I) dans lequel $R_2$ = cétyle ($C_{16}H_{33}$)

Dans 200cm³ de tétrahydrofuranne additionnés de 20cm³ d'eau, on solubilise à la température ambiante 16,9 g (0,05M) de chlorhydrate de dihydrosphingosine préparé à l'exemple 1. A cette solution on ajoute 15,2 g (0,05M) de cétylchloroformiate solubilisé dans 50cm³ de tétrahydrofuranne. A ce mélange réactionnel on ajoute 17 g (0,2M) d'hydrogénocarbonate de sodium et on maintient l'agitation à température ambiante toute la nuit. On ajoute alors 1 litre d'eau sous agitation. Le précipité formé est isolé par filtration sur verre fritté. On obtient après séchage 26,4 g d'un solide blanc de pF : 82°C répondant à la formule suivante :

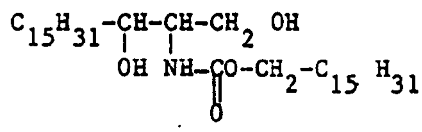

Le spectre RMN$^{13}$C confirme la structure attendue (mélange érythro-thréo)

Analyse élémentaire :

| | Calculé | mesuré |
|---|---|---|
| C | 73,76 | 73,10 |
| | | 73,09 |
| H | 12,56 | 12,60 |
| | | 12,63 |
| N | 2,46 | 2,40 |
| | | 2,40 |

EXEMPLE 3

1ère étape

Préparation du composé (II) avec $R_1$ = $C_{15}H_{31}$: 1,3-dihydroxy-2-amino-octadécane (forme érythro)

Le 2-acétamido-3-oxo-octadécanoate de méthyle (218 g, soit 0,59M) est mis en suspension dans 1090cm³ d'éthanol absolu. A 0°C, on ajoute une fois 65,4 g (1,73M) de borohydrure de sodium. On maintient l'agitation à cette température 3 heures, puis encore 4 heures à la température ambiante. Enfin, on chauffe 6 heures à reflux du solvant. Le solvant est alors éliminé sous pression réduite et le résidu est repris avec 600 cm³ d'acétate d'éthyle et 500 cm³ d'eau. On sépare les deux phases par décantation à 60°C. La phase supérieure est filtrée sur papier, puis abandonnée toute la nuit à 4°C.

On isole, par filtration sur verre fritté et après séchage, 178 g de solide blanc correspondant au 2-acétylamino-3-hydroxy octadécanol.

158 g du composé ci-dessus sont dissous dans 1054cm³ de méthanol à 10% d'eau contenant 129,1 g de potasse. Le milieu réactionnel est porté 6 heures à reflux puis filtré sur papier à chaud. Le filtrat est complété avec 300 cm³ d'eau et abandonné à 4°C pendant la nuit. Le solide formé est isolé par filtration sur verre fritté. On obtient ainsi 145 g de 2-amino-3-hydroxy-octadécanol ou dihydrosphingosine sous forme érythro pure.

Le spectre RMN$^{13}$C est conforme à la structure attendue :

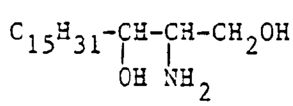

2ème étape

Préparation du composé (I) avec $R_2$ = phytyle ($C_{20}H_{39}$)

a) Préparation de l'imidazolide de phytol

Dans 180cm³ de dichlorométhane, on solubilise 18 g (0,11 M) de carbonyldiimidazole, puis on ajoute 29,6 g (0,1 M) de phytol solubilisé dans 30 cm³ du même solvant. Le mélange réactionnel est agité toute la nuit à la température ambiante. Après dilution du milieu avec 200 cm³ de dichlorométhane, on le lave à trois reprises avec 100cm³ d'eau. On sèche sur sulfate de sodium. Après élimination du solvant sous pression réduite, on isole 35,2 g d'imidazolide de phytol qui se présente sous la forme d'une huile.

Il répond à la formule ci-dessous :

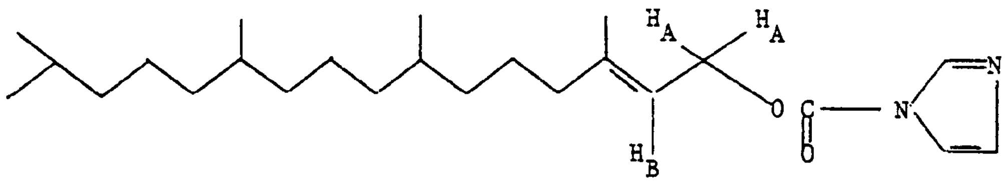

la structure est confirmée par le spectre RMN¹H.

b) Préparation du composé (I) avec $R_2$ = phytyle

Dans 100cm³ de tétrahydrofuranne, on solubilise 25 g (0,055M) d'imidazolide préparé ci-dessus que l'on ajoute à une solution du même solvant contenant 14,4 g (0,048M) de dihydrosphingosine préparée suivant la 1ère étape ci-dessus. Le milieu réactionnel est agité à la température ambiante toute la nuit. On élimine le solvant sous pression réduite. Le résidu obtenu est repris avec 300 cm³ de dichlorométhane et lavé à quatre reprises avec 100 cm³ d'eau.

Après séchage de la phase organique sur sulfate de sodium puis élimination du solvant sous pression réduite, on obtient un résidu qui est soumis successivement à deux séparations HPLC sur silice (KIESELGEL 60 de MERCK). La première séparation est effectuée avec le mélange dichlorométhane : méthanol = 98 : 2, la deuxième avec le mélange heptane : acétate d'éthyle = 6 : 4. On isole trois fractions A, B et C de poids respectifs 2, 1,5 et 2,5 g qui se présentent sous la forme de cires de pF<60°C.

Fraction A

Elle répond à la formule :

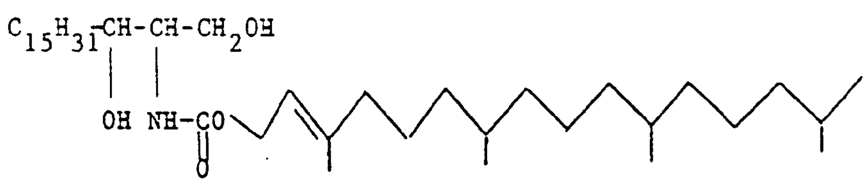

isomère trans

Analyse élémentaire :

| | Calculé | mesuré |
|---|---|---|
| C | 75,06 | 75,14 |
| H | 12,44 | 12,46 |
| N | 2,24 | 2,29 |

Le spectre RMN¹³C confirme la structure attendue

Fraction C

Elle répond à la formule :

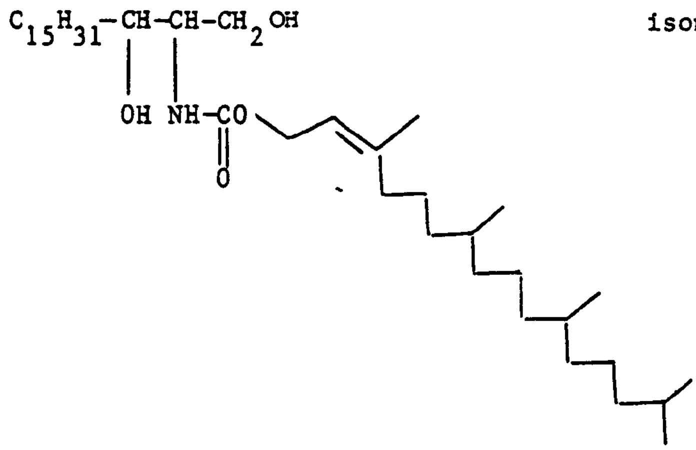

isomère cis

Le spectre RMN13C confirme la structure attendue.

Analyse élémentaire :

| | Calculé | mesuré |
|---|---|---|
| C | 75,06 | 75,08 |
| H | 12,44 | 12,47 |
| N | 2,24 | 2,17 |

Fraction B

Elle correspond d'après la chromatrographie sur couche mince et d'après la RMN au mélange des isomères cis et trans ci-dessus.

EXEMPLE 4

1ère étape

Préparation de l'imidazolide de 2-décyltétradécyle

Dans 105 cm³ de dichlorométhane, on solubilise 10,5 g (0,065 M) de carbonyldiimidazole. On ajoute ensuite à cette solution 20 g (0,057M) de 2-décyltétradécanol dilué dans 20cm³ de dichlorométhane. Le milieu réactionnel est abandonné toute la nuit à température ambiante sous agitation. On ajoute alors 200cm³ de dichlorométhane et on lave le milieu réactionnel à trois reprises avec 100 cm³ d'eau. Après concentration, on isole 26 g d'une huile, soit un rendement quantitatif en imidazolide de 2-décyltétradécyle répondant à la formule :

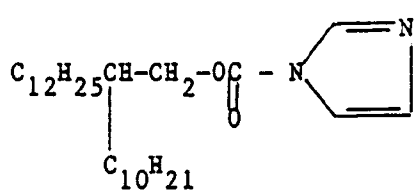

Le spectre RMN¹H confirme la structure attendue.

2ème étape

Préparation du composé (I) avec $R_2$ = 2-décyltétradécyle

Dans $100cm^3$ de tétrahydrofuranne, on solubilise 14,5 g (0,055M) d'imidazolide préparé ci-dessus que l'on ajoute à une solution du même solvant contenant 15,5 g (0,05M) de dihydrosphingosine préparée selon la 1ère étape de l'exemple 3. Le milieu réactionnel est agité à température ambiante toute la nuit, puis on évapore le solvant sous pression réduite. L'huile obtenue est soumise à une purification par HPLC sur une colonne de silice KIESELGEL 60 de MERCK avec comme éluant le mélange dichlorométhane : méthanol = 98 : 2.

On isole 13 g de solide blanc de pF : 72°C et répondant à la formule :

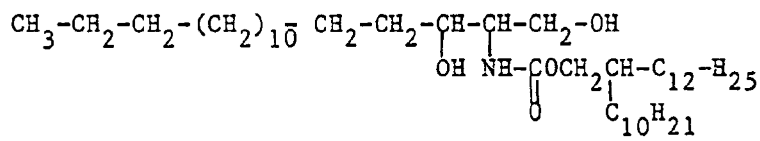

C' est l'isomère érythro pur.
Le spectre $RMN^{13}C$ confirme la structure attendue.

Analyse élémentaire :

| | Calculé | mesuré |
|---|---|---|
| C | 75,71 | 75,74 |
| H | 12,85 | 12,84 |
| N | 2,05 | 2,08 |

EXEMPLES D'APPLICATION

Exemple I

Crème de soins pour la peau

On a préparé une crème ayant la composition suivante :

- Composé de l'exemple 4 3 g
- Stéarate de glycérol 2 g
- TWEEN 60 (monostéarate de sorbitan à 20 moles d'oxyde d'éthylène) 1 g
- Alcool cétylique 0,5 g
- Acide stéarique 1,4 g
- Triéthanolamine 0,7 g
- CARBOPOL 940 (acide polyacrylique réticulé) neutralisé par la triéthanolamine 0,4 g
- Fraction liquide de graisse de karité 12 g
- Perhydrosqualène de synthèse 12 g
- Anti-oxydant 0,05 g
- Parfum 0,5 g
- Conservateur 0,3 g
- Eau qsp 100 g

Cette crème (émulsion huile-dans-l'eau) est préparée de la façon suivante :

on ajoute le CARBOPOL 940, neutralisé par la triéthanolamine à une partie de l'eau (85 à 90%) et on chauffe à 75-80°C. On ajoute alors en agitant la phase grasse (stéarate de glycérol, TWEEN 60, acide stéarique, composé de l'exemple 4, alcool cétylique, fraction liquide de graisse de karité, perhydrosqualène, anti-oxydant) portée à la même température, dans laquelle on a ajouté en dernier lieu la triéthanolamine. Après 10 minutes d'agitation, on ajoute le conservateur, dissous dans le restant de l'eau. Au bout de 10 minutes supplémentaires, on ajoute le parfum puis on arrête l'agitation et on refroidit jusqu'à la température ambiante (25°C).

Exemple II

Identique à l'exemple I, mais on remplace le composé de l'exemple 4 par 2 g de composé de l'exemple 3.

Exemple III

Lait corporel

On a préparé un lait ayant la composition suivante :
- Composé de l'exemple 3 3 g
- Monoisostéarate de sorbitan 5 g
- Cire microcristalline 1 g
- Huile de vaseline 15 g
- Huile de germes de maïs 4 g
- Mélange* d'esters d'acides gras en $C_8$-$C_{10}$ et d'alcools gras $C_{12}$-$C_{18}$ 1 g
- Gel** de montmorillonite modifié et d'huile neutre (triglycérides d'acides caprylique et caprique 5g
- Propylène glycol 3 g
- Anti-oxydant 0,1 g
- Conservateur 0,3 g
- Eau qsp 100 g

Ce lait corporel est préparé de façon analogue à cette décrite à l'exemple I.

Exemple IV

Crème hydratante

- Composé de l'exemple 1 5 g
- Stéarate de glycérol 2 g
- TWEEN 60 1 g
- Acide stéarique 1,4 g
- Triéthanolamine 0,7 g
- CARBOPOL 940 (neutralisé par la triéthanolamine) 0,2 g
- Huile d'amandes douces 3 g
- Huile de vaseline 8 g
- Anti-oxydant 0,05 g
- Conservateur 0,3 g
- Eau qsp 100 g

Cette crème est préparée de façon analogue à celle décrite à l'exemple I.

Exemple V

Dispersion hydratante

- Composé de l'exemple 2 ($R_2$ = cétyle) 1 g
- Cholestérol 1,5 g
- Dicétylphosphate de sodium 2,5 g
- Glycérol 3 g
- Conservateur 0,15 g
- Eau qsp 100 cc

Les lipides ainsi que le conservateur sont solubilisés dans 950 $cm^3$ d'un mélange de solvants dichlorométhane/méthanol (2/1). La solution est ensuite évaporée à sec. Sur le film lipidique on ajoute sous agitation, goutte à goutte, et en chauffant à 90°C, l'eau contenant le glycérol.

On obtient une dispersion de vésicules d'aspect laiteux, utilisée pour hydrater la peau.

* Commercialisé par HENKEL sous la dénomination commerciale "CETIOL-LC DEO"
** Commercialisé par DYNAMIT NOBEL sous la dénomination commerciale "MYGLYOL-GEL"

## Revendications

**1.** Composé lipidique ayant pour formule :

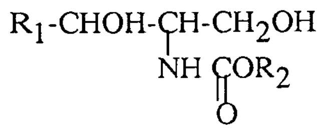

(I)

dans laquelle :

. $R_1$ désigne un radical alcoyle ou alcényle en $C_{11}$ à $C_{21}$ portant éventuellement un groupement hydroxyle et

. $R_2$ désigne un radical hydrocarboné, linéaire ou ramifié, en $C_8$ à $C_{30}$, saturé ou portant une ou plusieurs insaturations éthyléniques.

**2.** Composé selon la revendication 1, caractérisé par le fait que $R_1$ porte un groupement hydroxyle.

**3.** Composé selon la revendication 1, caractérisé par le fait que $R_2$ est un radical alcoyle ou alcényle.

**4.** Composé selon la revendication 1, caractérisé par le fait que $R_1$ désigne le radical pentadécyle et $R_2$ est choisi parmi les radicaux 2-éthylhexyle, cétyle, phytyle et 2-décyltétradécyle.

**5.** Procédé de préparation d'un composé de formule (I) selon l'une quelconque des revendications 1 à 4, caractérisé par le fait qu'il consiste à condenser sur la fonction amine d'une sphingosine, d'une dihydrosphingosine ou d'une phytosphingosine, un alcoyl- ou alcénylchloroformiate dans un solvant tel que le diméthylformamide ou le tétrahydrofuranne en présence d'une base telle que la triéthylamine ou la pyridine, ou un alcoyl- ou alcénylimidazolide isolé ou préparé in situ par réaction d'un alcool gras avec le carbonyldiimidazole, la réaction de condensation ayant lieu dans un solvant tel que le diméthylfomamide ou le tétrahydrofuranne.

**6.** Composition à usage cosmétique ou dermopharmaceutique, caractérisée par le fait qu'elle contient 0,05 à 20% en poids, de préférence 0,1 à 10% en poids, de composé de formule (I) selon la revendication 1, en présence d'un adjuvant choisi parmi les corps gras, les solvants, l'eau, les épaississants, les émulsionnants, les produits hydratants, les adoucissants, les filtres solaires, les germicides, les colorants, les conservateurs, les parfums, les propulseurs et les tensio-actifs.

**7.** Composition selon la revendication 6, caractérisée par le fait qu'elle se présente sous forme d'émulsion dont la phase grasse, représentant 5 à 60% du pois total de l'émulsion, est essentiellement constituée d'un mélange de composé de formule (I) avec au moins une huile, la phase aqueuse constituant 30 à 85% du poids total de l'émulsion, l'agent émulsionnant étant présent à raison de 1 à 20% en poids, et de préférence 2 à 12% en poids par rapport au poids total de l'émulsion.

**8.** Composition selon la revendication 6, caractérisée par le fait qu'elle se présente sous forme de lotion huileuse, oléoalcoolique ou hydroalcoolique, sous forme de gel, de dispersion ou de bâtonnets solides.

**9.** Composition selon la revendication 6, caractérisée par le fait qu'elle se présente sous forme d'une dispersion aqueuse de sphérules limidiques, constituées de couches moléculaires organisées renfermant une phase aqueuse encapsulée, ces couches étant constituées d'au moins un composé de formule (I) dans laquelle $R_2$ représente une chaîne linéaire, associé à au moins un autre composé lipidique.

**10.** Composition sous forme de dispersion aqueuse de sphérules lipidiques selon la revendication 9, caractérisée par le fait que l'autre composé lipidique est choisi parmi les alcools et diols à longue chaîne, les stérols, les phospholipides, les glycolipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras, les alkylsulfates.

**11.** Composition sous forme de dispersion aqueuse de sphérules lipidiques selon la revendication 9, caractérisée par le fait que les sphérules ont un diamètre compris entre 0,1μm et 5μm.

**12.** Composition sous forme de dispersion aqueuse de sphérules lipidiques selon la revendication 9, caractérisée par le fait que la phase aqueuse encapsulée dans les sphérules est de l'eau ou une solution aqueuse de substance active.

**13.** Composition sous forme de dispersion aqueuse de sphérules lipidiques selon l'une quelconque des revendications 9 à 12, caractérisée par le fait que la phase aqueuse encapsulée dans les sphérules contient au moins une substance active cosmétique choisie parmi les humectants, les agents de brunissage artificiel éventuellement associés à des colorants, les filtres solaires hydrosolubles, les antiperspirants, les déodorants, les astringents, les produits rafraîchissants, les toniques les cicatrisants, les kératolytiques, les dépilatoires, les eaux parfumées, les extraits de tissus animaux ou végétaux, les colorants hydrosolubles, les agents anti-pelliculaires, les agents anti-séborrhéiques, les oxydants et les réducteurs.

**14.** Composition sous forme de dispersion aqueuse de sphérules lipidiques selon l'une quelconque des revendications 9 à 12 caractérisée par le fait que la phase aqueuse encapsulée dans les sphérules contient au moins une substance active choisie parmi les vitamines, les hormones, les enzymes, les vaccins, les anti-inflammatoires, les antibiotiques, les bactéricides et les agents cytotoxiques ou antitumoraux.

**15.** Utilisation du composé de formule (I) selon l'une quelconque des revendications 1 à 4, en tant que constituant cireux ayant des propriétés émollientes et adoucissantes dans des émulsions, des dispersions, des gels, des bâtonnets solides ou dans des lotions à usage cosmétique ou dermopharmaceutique.

**16.** Utilisation du composé lipidique de formule (I) dans laquelle $R_2$ représente un chaîne linéaire, selon l'une quelconque des revendications 1 à 4, en association avec au moins un autre composé lipidique, pour la formation de dispersions de sphérules lipidiques à usage cosmétique ou dermopharmaceutique.

**17.** Procédé de traitement cosmétique de la peau ou des cheveux, caractérisé par le fait qu'il consiste à appliquer sur la peau ou les cheveux une quantité suffisante d'une composition selon l'une quelconque des revendications 6 à 13.

## Patentansprüche

**1.** Lipidverbindung der Formel:

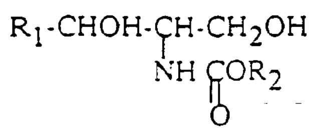

(I)

worin:

- $R_1$ einen gegebenenfalls eine Hydroxylgruppe aufweisenden $C_{11-21}$-Alkyl- oder -Alkenylrest und
- $R_2$ einen linearen oder verzweigten gesättigten oder ein- oder mehrfach ethylenisch ungesättigten $C_{8-30}$-Kohlenwasserstoffrest bedeuten.

**2.** Verbindung gemäß Anspruch 1,
dadurch **gekennzeichnet**, daß
$R_1$ eine Hydroxylgruppe aufweist.

**3.** Verbindung gemäß Anspruch 1,
dadurch **gekennzeichnet**, daß
$R_2$ ein Alkyl- oder Alkenylrest ist.

**4.** Verbindung gemäß Anspruch 1,
dadurch **gekennzeichnet**, daß
$R_1$ den Pentadecylrest bedeutet und $R_2$ aus 2-Ethylhexyl-, Cetyl-, Phytyl- und 2-Decyltetradecylresten ausgewählt ist.

5. Verfahren zur Herstellung einer Verbindung der Formel (I) gemäß jedem der Ansprüche 1 bis 4, dadurch **gekennzeichnet**, daß man an der Aminfunktion eines Sphingosins, Dihydrosphingosins oder Phytosphingosins, eine Kondensationsreaktion mit einem Alkyl- oder Alkenylchlorformiat in einem Lösungsmittel wie Dimethylformamid oder Tetrahydrofuran in Gegenwart einer Base wie Triethylamin oder Pyridin oder mit einem Alkyl- oder Alkenyloxicarboxyimidazolid durchführt, das man isoliert oder in situ durch Reaktion mit einem Fettalkohol mit Carbonyldiimidazol herstellt, wobei die Kondensationsreaktion in einem Lösungsmittel wie Dimethylformamid oder Tetrahydrofuran stattfindet.

6. Zusammensetzung zur kosmetischen oder dermopharmazeutischen Verwendung, dadurch **gekennzeichnet,** daß sie 0,05 bis 20 Gew.%, vorzugsweise 0,1 bis 10 Gew.%, der Verbindung der Formel (I) gemäß Anspruch 1 in Gegenwart eines Hilfsstoffes enthält, ausgewählt unter Fettkörpern, Lösungsmitteln, Wasser, Verdickungsmitteln, Emulgatoren, Hydratisierprodukten, Weichmachern, Sonnenfilterstoffen, Germiziden, Färbemitteln, Konservierungsstoffen, Parfüms, Treibmitteln und oberflächenaktiven Mitteln.

7. Zusammensetzung gemäß Anspruch 6, dadurch **gekennzeichnet**, daß sie in Form einer Emulsion vorliegt, deren Fettphase, die 5 bis 60% des Gesamtgewichts der Emulsion ausmacht, im wesentlichen aus einer Mischung der Verbindung der Formel (I) mit mindestens einem Öl zusammengesetzt ist, wobei die wässrige Phase 3 bis 85% des Gesamtgewichts der Emulsion ausmacht und der Emulgator in einer Menge von 1 bis 20 Gew.%, vorzugsweise 2 bis 12 Gew.%, bezogen auf Gesamtgewicht der Emulsion, vorliegt.

8. Zusammensetzung gemäß Anspruch 6, dadurch **gekennzeichnet**, daß sie in Form einer öligen, oleoalkoholischen oder hydroalkoholischen Lotion oder in Form eines Gels, einer Dispersion oder von Feststoffstäbchen vorliegt.

9. Zusammensetzung gemäß Anspruch 6, dadurch **gekennzeichnet**, daß sie in Form einer wässrigen Dispersion lipidischer Kügelchen vorliegt, die aus molekularen organischen Schichten aufgebaut sind, welche eine wässrige Phase eingekapselt enthalten, wobei diese Schchten aus mindestens einer Verbindung der Formel (I), worin $R_2$ eine lineare Kette darstellt, zusammen mit mindestens einer weiteren Lipidverbindung aufgebaut sind.

10. Zusammensetzung in Form einer wässrigen Dispersion lipidischer Kügelchen gemäß Ansrpuch 9, dadurch **gekennzeichnet**, daß die weitere Lipidverbindung aus langkettigen Alkoholen und Diolen, Sterolen, Phospholipiden, Glycolipiden, Cholesterylsulfat und -phosphat, langkettigen Aminen und deren quaternären Ammoniumderivaten, Dihydroxyalkylaminen, polyoxethylierten Fettaminen, langkettigen Aminoalkoholestern, deren Salzen und quaternären Ammoniumderivaten, Phosphorsäureestern von Fettalkoholen und Alkylsulfaten ausgewählt ist.

11. Zusammensetzung in Form einer wässrigen Dispersion lipidischer Kügelchen gemäß Anspruch 9, dadurch **gekennzeichnet**, daß die Kügelchen einen Durchmesser von 0,1 bis 5 μm aufweisen.

12. Zusammensetzung in Form einer wässrigen Dispersion lipidischer Kügelchen gemäß Anspruch 9, dadurch **gekennzeichnet**, daß die in die Kügelchen eingekapselte wässrige Phase Wasser oder eine wässrige Lösung einer Wirksubstanz ist.

13. Zusammensetzung in Form einer wässrigen Dispersion lipidischer Kügelchen gemäß jedem Anspruch 9 bis 12, dadurch **gekennzeichnet**, daß die in den Kügelchen eingekapselte wässrige Phase mindestens eine kosmetisch wirksame Substanz enthält, ausgewählt aus Feuchtigkeitsmitteln, künstlichen Bräunungsmitteln, gegebenenfalls zusammen mit Färbemitteln, wasserlöslichen Sonnenfilterstoffen, Antischweißmitteln, Deodoriermitteln, Hautfestigungsmitteln, Erfrischungsprodukten, tonischen Mitteln, Vernarbungsmitteln, keratolytischen Mitteln, Enthaarungsmitteln, parfümierten Wässern, Extrakten tierischer oder pflanzlicher Gewebe, wasserlöslichen Färbemitteln, anti-filmbildenden Mitteln, anti-seborrheischen Mitteln, Oxidations- und Reduktionsmitteln.

**14.** Zusammensetzung in Form einer wässrigen Dispersion lipidischer Kügelchen gemäß jedem Anspruch 9 bis 12,
dadurch **gekennzeichnet**, daß
die in den Kügelchen eingekapselte wässrige Phase mindestens eine Wirksubstanz enthält, ausgewählt aus Vitaminen, Hormonen, Enzymen, Impfstoffen, entzündungshemmenden Mitteln, Antibiotika, Bakteriziden und zytotoxischen oder antitumoralen Mitteln.

**15.** Verwendung der Verbindung der Formel (I) gemäß jedem Anspruch 1 bis 4 als wachsartigen Bestandteil mit weichmachenden und glättenden Eigenschaften in Emulsionen, Dispersionen, Gelen, Feststoffstäbchen oder in Lotionen zur kosmetischen oder dermopharmazeutischen Verwendung.

**16.** Verwendung der Lipidverbindung der Formel (I), worin $R_2$ eine lineare Kette darstellt, gemäß jedem Anspruch 1 bis 4, zusammen mit mindestens einer weiteren Lipidverbindung, zur Bildung von Dispersionen lipidischer Kügelchen zur kosmetischen oder dermopharmazeutischen Verwendung.

**17.** Verfahren zur kosmetischen Behandlung der Haut oder der Haare,
dadurch **gekennzeichnet**, daß
man auf die Haut oder die Haare eine ausreichende Menge einer Zusammensetzung gemäß jedem Anspruch 6 bis 13 aufträgt.

## Claims

**1.** Lipid compound having the formula:

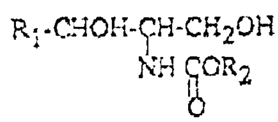

(I)

in which:
- $R_1$ denotes a $C_{11}$ to $C_{21}$ alkyl or alkenyl radical optionally bearing a hydroxyl group, and
- $R_2$ denotes a linear or branched $C_8$ to $C_{30}$ hydrocarbon radical, saturated or bearing one or more ethylenic unsaturations.

**2.** Compound according to Claim 1, characterized in that $R_1$ bears a hydroxyl group.

**3.** Compound according to Claim 1, characterized in that $R_2$ is an alkyl or alkenyl radical.

**4.** Compound according to Claim 1, characterized in that $R_1$ denotes a pentadecyl radical and $R_2$ is chosen from 2-ethylhexyl, cetyl, phytyl and 2-decyltetradecyl radicals.

**5.** Process for preparing a compound of formula (I) according to any one of Claims 1 to 4, characterized in that it consists in condensing the amine function of a sphingosine, dihydrosphingosine or phytosphingosine with an alkyl or alkenyl chloroformate in a solvent such as dimethylformamide or tetrahydrofuran in the presence of a base such as triethylamine or pyridine, or with an alkyl- or alkenylimidazolide, isolated or prepared in situ by reacting a fatty alcohol with carbonyldiimidazole, the condensation reaction taking place in a solvent such as dimethylformamide or tetrahydrofuran.

**6.** Composition for cosmetic or dermopharmaceutical use characterized in that it contains 0.05 to 20 % by weight, and preferably 0.1 to 10 % by weight, of compound of formula (I) according to Claim 1, in the presence of an adjuvant chosen from fats, solvents, water, thickeners, emulsifiers, hydrating products, demulcents, sunscreen agents, germicides, colorants, preservatives, perfumes, propellants and surfactants.

**7.** Composition according to Claim 6, characterized in that it takes the form of an emulsion in which the fatty

phase, representing 5 to 60 % of the total weight of the emulsion, essentially consists of a mixture of compound of formula (I) with at least one oil, the aqueous phase constituting 30 to 85 % of the total weight of the emulsion, the emulsifying agent being present in the proportion of 1 to 20 % by weight, and preferably 2 to 12 % by weight, relative to the total weight of the emulsion.

**8.** Composition according to Claim 6, characterized in that it takes the form of an oily, oleoalcoholic or aqueous-alcoholic lotion or the form of a gel, dispersion or solid sticks.

**9.** Composition according to Claim 6, characterized in that it takes the form of an aqueous dispersion of lipid spherules consisting of organized molecular layers containing an encapsulated aqueous phase, these layers consisting of at least one compound of formula (I) in which $R_2$ represents a linear chain, combined with at least one other lipid compound.

**10.** Composition in the form of an aqueous dispersion of lipid spherules according to Claim 9, characterized in that the other lipid compound is chosen from long-chain alcohols and diols, sterols, phospholipids, glycolipids, cholesteryl sulphate and phosphate, long-chain amines and their quaternary ammonium derivatives, dihydroxyalkylamines, polyoxyethylenated fatty amines, esters of long-chain amino alcohols, their salts and quaternary ammonium derivatives, phosphoric esters of fatty alcohols and alkyl sulphates.

**11.** Composition in the form of an aqueous dispersion of lipid spherules according to Claim 9, characterized in that the spherules are between 0.1 μm and 5 μm in diameter.

**12.** Composition in the form of an aqueous dispersion of lipid spherules according to Claim 9, characterized in that the aqueous phase encapsulated in the spherules is water or an aqueous solution of active substance.

**13.** Composition in the form of an aqueous dispersion of lipid spherules according to any one of Claims 9 to 12, characterized in that the aqueous phase encapsulated in the spherules contains at least one cosmetic active substance chosen from humectants, artificial tanning agents optionally combined with colorants, water-soluble sunscreen agents, antiperspirants, deodorants, astringents, freshening products, tonics, healing agents, keratolytics, depilatories, scented waters, animal or plant tissue extracts, water-soluble colorants, antidandruff agents, antiseborrhoeic agents and oxidizing and reducing agents.

**14.** Composition in the form of an aqueous dispersion of lipid spherules according to any one of Claims 9 to 12, characterized in that the aqueous phase encapsulated in the spherules contains at least one active substance chosen from vitamins, hormones, enzymes, vaccines, anti-inflammatories, antibiotics, bactericides and cytotoxic or antitumour agents.

**15.** Use of the compound of formula (I) according to any one of Claims 1 to 4, as a waxy constituent having emollient and demulcent properties in emulsions, dispersions, gels or solid sticks or in lotions for cosmetic or dermopharmaceutical use.

**16.** Use of the lipid compound of formula (I) in which $R_2$ represents a linear chain, according to any one of Claims 1 to 4, in combination with at least one other lipid compound, for the formation of dispersions of lipid spherules for cosmetic or dermopharmaceutical use.

**17.** Process for cosmetic treatment of the skin or hair, characterized in that it consists in applying to the skin or hair a sufficient amount of a composition according to any one of Claims 6 to 13.